(19) Europäisches Patentamt
European Patent Office
Office européen des brevets

(11) Veröffentlichungsnummer: **0 254 243**
**A2**

(12) # EUROPÄISCHE PATENTANMELDUNG

(21) Anmeldenummer: **87110437.8**

(22) Anmeldetag: **18.07.87**

(51) Int. Cl.³: **C 07 C 69/16**
**C 12 P 7/62**

(30) Priorität: **22.07.86 DE 3624703**

(43) Veröffentlichungstag der Anmeldung:
**27.01.88 Patentblatt 88/4**

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Schneider, Manfred, Prof. Dr.**
**Triebelsheider Weg 37**
**D-5600 Wuppertal(DE)**

(72) Erfinder: **Laumen, Kurt**
**Arrenbergerstrasse 41**
**D-5600 Wuppertal(DE)**

(72) Erfinder: **Breitgoff, Detlef**
**Wilhelmring 19**
**D-5600 Wuppertal(DE)**

(72) Erfinder: **Wullbrandt, Dieter, Dr.**
**Bienerstrasse 29**
**D-6238 Hofheim am Taunus(DE)**

(72) Erfinder: **Schlingmann, Merten, Dr.**
**Schneidhainer Strasse 32a**
**D-6240 Königstein/Taunus(DE)**

(72) Erfinder: **Keller, Reinhold, Dr.**
**Wiesenweg 5**
**D-6232 Bad Soden am Taunus(DE)**

(54) **Chirale Synthesebausteine aus prochiralem Glycerin.**

(57) Durch Differenzierung der beiden enantiotopen Gruppen in prochiralen Glycerinderivaten gelangt man direkt zu chiralen Molekülen, die wiederum leicht durch selektive Manipulation der funktionellen Gruppen sowohl in die S- als auch in die R-Reihe übergeführt werden.

EP 0 254 243 A2

Croydon Printing Company Ltd.

Beschreibung



Chirale Synthesebausteine aus prochiralem Glycerin

Chirale Glycerinderivate, beispielsweise (S)- und (R)-2,2-Dimethyl-1,3-dioxolan-4-methanole 1 oder die entsprechenden Aldehyde (R) und (S) (2) sind in der Vergangenheit intensiv als Bausteine für die Synthese enantiomerenreiner Natur- und Wirkstoffe verwendet worden. Beispiele dafür sind die Herstellung von Phospholipiden, PAF (platelet aggregation factor), ß-Blockern, (-)-γ-Amino-ß-hydroxybuttersäure (GABOB), Prostaglandinen, Brefeldin A und vielen anderen.

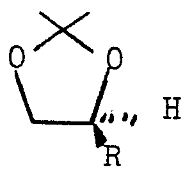

(S) 1; $R=CH_2OH$

(R) 2; R=CHO

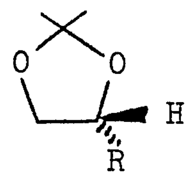

(R) 1; $R=CH_2OH$

(S) 2; R=CHO

1 und 2 können in eine Vielzahl wichtiger Zwischenprodukte übergeführt werden, die als Ausgangsmaterialien für die Herstellung von Lipiden, Kohlenhydraten, Nucleotidanaloga, Naturstoffen und vielen anderen, optisch aktiven Molekülen Verwendung finden können.

Enantiomerenreine, chirale Bausteine mit Glyceringrundgerüst werden bisher durch Synthese aus Monosacchariden und Aminosäure gewonnen, wie aus den folgenden Literaturzitaten zu entnehmen ist: G. Wirk, W. Walter: Helv. Qim. Acta, 68, 1963 (1985); C.M. Lok et al.: Chem. Phys. Lipids 16, 115 (1976); H.O.L. Fischer, E. Baer: J. Biol. Chem. 128, 463 (1939).

Glycerin ist bislang nicht als alternative Quelle für derartige Synthesebausteine in Erwägung gezogen worden. In Anbetracht großer Mengen von Lipiden in Fetten und Ölen,

die aus nachwachsender Biomasse zur Verfügung stehen, ist ein direkter Weg von Glycerin über eine prochirale Zwischenstufe zu chiralen Synthesebausteinen von beträchtlichem Interesse.

Es wurde überraschend gefunden, daß man durch Differenzierung der beiden enantiotopen Gruppen in prochiralen Glycerinderivaten direkt zu chiralen Molekülen gelangt, die wiederum leicht durch selektive Manipulation der funktionellen Gruppen sowohl in die S- als auch in die R-Reihe übergeführt werden können.

Die Erfindung betrifft somit:

1. Die R- und S-Enantiomere der Verbindung der allgemeinen Formel I,

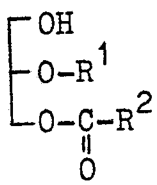

I

in der $R^1$ eine Ether-bildende Schutzgruppe und $R^2$ eine verzweigte oder vorzugsweise geradkettige Alkylgruppe mit 1 bis 18 Kohlenstoffatomen darstellt, die gegebenenfalls durch Halogen, Hydroxy, Alkoxy mit 1 bis 3 Kohlenstoffatomen, Phenyl, Phenoxy und/oder Thienyl substituiert sein kann, wobei eine Phenyl- oder Phenoxygruppe substituiert sein kann durch Alkyl, Amin, Hydroxy, Halogen und Alkoxy.

2. Ein Verfahren zur Herstellung der R- oder S-Enantiomeren der Verbindung der obengenannten allgemeinen Formel I, das dadurch gekennzeichnet ist, daß aus der Verbindung der allgemeinen Formel II

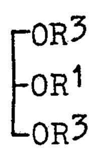

II

in der $R^1$ die obengenannte Bedeutung hat und $R^3$ Wasserstoff oder $R^2-\overset{O}{\overset{\|}{C}}-$ ist, wobei $R^2$ ebenfalls die obengenannte Bedeutung hat,

durch Inkubation mit Hydrolasen,

wenn $R^3$ Wasserstoff ist, mit der Verbindung der allgemeinen Formel $R^2-\overset{O}{\overset{\|}{C}}-OR^4$, in der $R^2$ die obengenannte Bedeutung hat und $R^4$ $C_1$ bis $C_6$ Alkyl oder Alkenyl, geradkettig oder verzweigt, das durch Halogen, Hydroxy, Alkoxy und Nitro substituiert sein kann, bedeutet, selektiv eine Hydroxylgruppe verestert wird oder,

wenn $R^3$ die Gruppe $R^2-\overset{O}{\overset{\|}{C}}-$ bedeutet, eine Estergruppe selektiv abgespalten wird.

Im folgenden wird die Erfindung näher erläutert bzw. in den Ansprüchen definiert.

Die Verbindung der allgemeinen Formel II ist ein Zwischenprodukt bei der Herstellung der Verbindung der allgemeinen Formel I. Die Ausgangsverbindung der allgemeinen Formel II zur Herstellung der Verbindung I kann leicht nach an sich bekannten Verfahren N. Bagett el al., Chem. Soc. 2574 (1960) hergestellt werden. Durch selektive enzymatische Verseifung mit Hilfe von Hydrolasen wird die Verbindung der allgemeinen Formel I aus der Verbindung der allgemeinen Formel II gewonnen. Dabei soll nur eine der beiden Acyl-Gruppen abgespalten werden, so daß keine unerwünschten Nebenprodukte entstehen. $R^1$ bedeutet sowohl in der Verbindung der allgemeinen Formel I als auch in der Verbindung der allgemeinen Formel II eine Ether-bildende Schutzgruppe. Geeignet sind Methylether, die gegebenenfalls substituiert sein können, wie beispielsweise Methoxymethyl, 2-Methoxyethoxymethyl und Tetrahydropyranyl,

oder substituierte Ethylether, wie beispielsweise Benzyl, p-Methoxybenzyl, o-Nitrobenzyl, p-Nitrobenzyl, p-Halobenzyl, p-Cyanobenzyl und Triphenylmethyl, oder Silylether, wie beispielsweise Trimethylsilyl und t-Butyldiphenylsilyl.

Bevorzugt wird für $R^1$ Benzyl bzw. substituiertes Benzyl als Schutzgruppe verwendet.

Die Schutzgruppe $R^2$ stellt eine verzweigte und vorzugsweise geradkettige Alkylgruppe mit 1 bis 18 C-Atomen, bevorzugt 1 bis 5 C-Atomen, dar, die gegebenenfalls durch Halogen, Hydroxy, Alkoxy mit 1 bis 3 C-Atomen, Phenyl, Phenoxy und/oder Thienyl substituiert sein kann, wobei eine Phenyl- oder Phenoxygruppe substituiert sein kann durch Alkyl, Amin, Hydroxy, Halogen und Alkoxy.

In Abhängigkeit von der Selektivität des Enzyms kann die R- oder S-Konfiguration der Verbindung der allgemeinen Formel I gebildet werden. Geeignet sind unter anderem Esterase bzw. Lipase aus verschiedenen Mikroorganismen, beispielsweise aus Candida cylindracea, Mucor sp. oder Chromobacterium viscosum, oder auch aus Schweineleber oder Schweinepankreas. Besonders bevorzugt sind Lipase aus Schweinepankreas (E.C. 3.1.1.3) bzw. aus Pseudomonas spec., wie z.B. Lipoproteinlipase (E.C. 3.1.1.34). Die Enzyme können sowohl in löslicher Form als auch an Träger fixiert entsprechend dem Stand der Technik eingesetzt werden [EP 220593; Laumen et al., Tetrahedron Letters 26, 407 (1985)].

Die Lipoproteinlipase spaltet im wesentlichen nur eine Acylgruppe enantioselektiv ab. Man erhält ein Produkt von hoher Enantiomerenreinheit. Wenn Lipase aus Schweinepankreas zur Hydrolyse der Verbindung der allgemeinen Formel II verwendet wird, in

der $R^3$ die Gruppe $R^2-\overset{O}{\overset{\|}{C}}-$ bedeutet, ist die Selektivität sowie die Bildung des Nebenprodukts, in dem beide Estergruppen abgespalten sind, abhängig von der Substratkonzentration sowie dem Verhältnis von Substrat zu Enzym. Bei der Verwendung höherer Enzym- und Substratkonzentrationen erreicht man in wenigen Stunden einen hohen Umsatz zu dem Produkt I mit hoher Enantiomerenreinheit. Durch eine Erhöhung der Löslichkeit der Verbindung der allgemeinen Formel II in dem wäßrigen Reaktionsmedium unter Zugabe von Lösemitteln, die bedingt oder vollständig mit Wasser mischbar sind die Enzymaktivität aber nicht wesentlich vermindern oder seine Spezifität verändern, kann die Reaktionsgeschwindigkeit erhöht werden. Als Lösemittel eignen sich cyclische Ether wie Tetrahydrofuran und Dioxan $(C_1-C_3)$-Alkanole, Aceton oder Dimethylsufoxid oder Dimethylformamid.

Die Reaktion läuft bei 10 bis 50°C, bevorzugt 20 bis 35°C ab. Bei höheren Temperaturen werden die Enzyme zunehmend desaktiviert, es sei denn, es handelt sich um thermophile Proteine. Bei niedrigeren Temperaturen läuft die Reaktion zu langsam ab. Der pH- Wert liegt zwischen 5 und 8, bevorzugt zwischen 6,5 und 7,5. Die Enzymkonzentration kann in weiten Bereichen schwanken, insbesondere, da sie, wie oben erwähnt, in Abhängigkeit von der Substratkonzentration gewählt werden kann. Zweckmäßig wählt man bei einer Substratkonzentration von 0,05 bis 2,5 Mol/l, bevorzugt 0,2 bis 1,5 Mol/l eine Enzymkonzentration von 500 bis 20000 U/mMol Substrat, vorzugsweise 600 bis 8000 U/mMol Substrat. Die Lipoproteinlipase kann in geringeren Mengen, beispielsweise 10 bis 2000 U/mMol Substrat, eingesetzt werden. Ein Unit (U) der Enzymaktivität bezeichnet die Menge, die pro Minute bei pH 7,0 und einer Temperatur von 37°C, aus einem Triglycerid 1,0 Mikroäquivalent Fettsäure hydrolytisch abspaltet. Die optimalen Reaktionsbedindungen

sind natürlich davon abhängig, welches Enzym eingesetzt wird. Sie sind jedoch leicht vom Fachmann zu ermitteln.

Mit Hilfe des erfindungsgemäßen Verfahrens kann wie erwähnt nicht nur die Hydrolyse durchgeführt werden, sondern bei sehr niedrigen Wasserkonzentrationen von ca. 0,1 bis 5 % $H_2O$, bevorzugt 0,25-1,5 %, bezogen auf das Gewicht des Reaktionsansatzes, auch die Alkoholyse der Verbindung der allgemeinen Formel II, in der $R^3$ Wasserstoff bedeutet. Die Reaktionsbedingungen der Alkoholyse gleichen ansonsten denen der Hydrolyse. Da sowohl bei der Hydrolyse als auch bei der Alkoholyse vom Enzym die gleiche enantiotope Gruppe erkannt wird, ergeben sich bei der Verwendung prochiraler Substrate jeweils die entgegengesetzten absoluten Konfigurationen, wie z.B. in der folgenden Abbildung dargestellt.

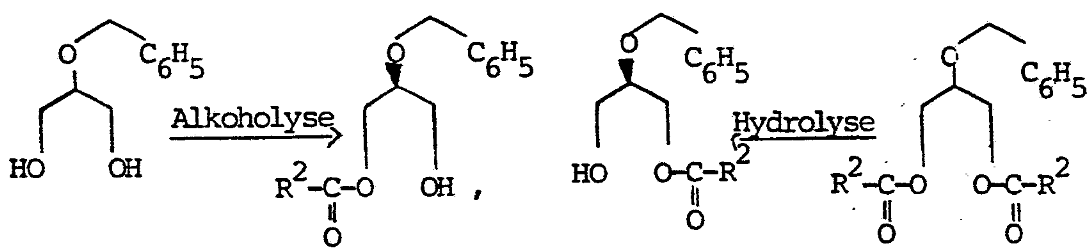


S R

Die Verbindung der allgemeinen Formel II ist das Zwischenprodukt und damit Schlüsselsubstanz für die Herstellung weiterer Verbindungen auf dem Wege zu pharmazeutisch aktiven Substanzen, wie beispielsweise oben aufgeführt. Diese erhält man durch Umsetzung der Verbindung I mit Reagenzien, die sich als Schutzgruppe für die Hydroxylgruppe eignen und die sich wieder abspalten lassen. Bevorzugte Schutzgruppen liefern Dihydropyran, Tritylhalogenid, Toluolsulfonsäurehalogenid, trisubstituiertes Silylhalogenid, Methoxymethylhalogenid oder 2-Methoxyethoxymethylhalogenid. Die Reaktionsbedingungen, unter

denen Schutzgruppen eingeführt werden bzw. wieder abgespalten werden können, sind der entsprechenden Literatur zu entnehmen, beispielsweise: Protective Groups in Organic Synthesis T.W. Greene (Wiley Intersciences, 1981) oder J.F.W. McOmie (Plenum Press, London, 1973).

Durch anschließende Abspaltung der $R^2CO$-Gruppe erhält man die entsprechenden Verbindungen mit einer freien Hydroxylgruppe, die wiederum Ausgangsprodukt für obengenannte pharmazeutische Verbindungen sind.

Die Reaktionsbedingungen, unter denen die $R^2CO$-Gruppe abgespalten werden können, sind literaturbekannt. Man arbeitet beispielsweise in einem schwach basischen Medium mit einem Alkohol als Lösungsmittel ($K_2CO_3$/Methanol, $NH_3$/Methanol, NaOH/Pyridin oder Ethanol) oder im sauren Bereich (HCl/Aceton).

Durch die oben beschriebenen selektiven Manipulationen am Zwischenprodukt I, können, in Abhängigkeit von deren Konfiguration, Synthesebausteine beider enantiomerer Reihen hergestellt werden. Geht man beispielsweise von dem R-Enantiomeren der Verbindung I aus, so erhält man, je nachdem welche Schutzgruppe eingeführt wird, das R- oder S-Enantiomer der daraus resultierenden Verbindungen. Spaltet man dann die $R^2CO$-Gruppen ab, so erhält man in allen Fällen das S-Enantiomer der resultierenden Hydroxylverbindung. Diese S-Hydroxylverbindung kann wiederum mit entsprechenden Schutzgruppen liefernden Reagenzien umgesetzt werden, wodurch man nach Abspaltung der ursprünglichen Schutzgruppen zu den R-Hydroxylverbindungen gelangt. Das Formelschema auf S.19 soll diesen Sachverhalt beispielhaft erläutern.

Der große synthetische Nutzen der Verbindung II wird durch seine leichte Abwandelbarkeit in die R- oder S-Reihe

deutlich. Eine Vielzahl nützlicher chiraler Glycerinderivate beider enantiomerer Reihen können mit Hilfe dieser Zwischenprodukte hergestellt werden. Mit einfachem Glycerin als Ausgangsmaterial wurde hier zum ersten Mal ein alternativer, chemoenzymatischer Weg zu derartigen Synthesebausteinen erschlossen. In Anbetracht großer Mengen von Lipiden, insbesondere Fette und Öle, aus nachwachsender Biomasse eröffnet sich durch diese grundlegende Arbeit ein wirtschaftlicher Zugang zu wichtigen Bausteinen der Natur- und Wirkstoffsynthese.

In den nachfolgenden Beispielen wird die Erfindung detailliert erläutert. Prozentangaben beziehen sich, wenn nicht anders angegeben, auf das Gewicht.

Beispiele

Enzymatische Hydrolyse von 2-O-Benzylglycerin-1,3-diacetat.

1. 50 m Mol (13,3 g) 2-O-Benzylglycerin-1,3-diacetat werden in 100 ml 0,1 M Phosphatpuffer (pH 7, 20°C) suspendiert und mit 200 mg Lipase aus Schweinepankreas (PPL, EC 3.1.1.3) versetzt. Durch kontinuierliche Zugabe von 1 N NaOH aus einer Autobürette wird der pH-Wert konstant auf pH 7 gehalten. Die Hydrolyse wird nach einem Verbrauch von 50 ml 1N NaOH (ca. 4 h) abgebrochen und das Reaktionsgemisch kontinuierlich mit 150 ml Ether extrahiert (ca. 12 h). Die organische Phase wird über $MgSO_4$ getrocknet, das Lösungsmittel abgezogen und der Rückstand an Kieselgel 60 (70 - 230 mesh, Diethylether/Hexan 1 : 2) chromatographiert.

| | | Rf |
|---|---|---|
| Fraktion 1: | 2-O-Benzylglycerin-1,3-diacetat | 0,7 |
| " 2: | R-1-Hydroxy-2-O-benzylglycerin-3-acetat | 0,4 |
| " 3: | 2-O-Benzylglycerin | |

Ausbeute von R-1-Hydroxy-2-O-benzylglycerin-3-acetat: 5,3 g (47 %)

Optischer Drehwert: $[\alpha]_D^{20}$ - 8,98° (c 2,78 Ethanol);
$[\alpha]_D^{20}$ + 12,75°,
$[\alpha]_{365}^{20}$ + 40,5° (c 1,796 Chloroform + 1 % Ethanol);

Optische Reinheit: 60 % e.e.

2. 100 m Mol (26,6 g) 2-O-Benzylglycerin-1,3-diacetat werden in 200 ml eines Gemisches aus 175 ml 0,1 M Phosphatpuffer (pH 7, T= 20°C) und 25 ml Tetrahydrofuran suspendiert und mit 250 mg Lipase aus Schweinepankreas (PPL., E.C. 3.1.1.3) versetzt. Durch kontinuierliche Zugabe von 1N NaOH aus einer Autobürette wird der pH-Wert konstant auf pH 7 gehalten. Die Hydrolyse wird nach einem Verbrauch von 96 ml 1N NaOH (4 h) abgebrochen und das Reaktionsgemisch kontinuierlich mit 150 ml Diethylether extrahiert. Die organische Phase wird über $MgSO_4$ getrocknet, das Lösungsmittel abgezogen und der Rückstand über Kieselgel 60 (70 - 230 mesh, Diethylether/Hexan 1 : 2) chromatographiert.

Ausbeute von R-1-Hydroxy-2-O-benzylglycerin-3-acetat: 8,7 g (40 %)

Optischer Drehwert: $[\alpha]_D^{20}$ - 12°,
$[\alpha]_{365}^{20}$ - 40,3° (c 2,99 in Ethanol);
$[\alpha]_D^{20}$ + 16,75°,
$[\alpha]_{365}^{20}$ + 52,6° (c 1,74 Chloroform + 1 % Ethanol);

Optische Reinheit: 84 % e.e.

3. 150 m Mol (40 g) 2-O-Benzylglycerin-1,3-diacetat werden in 150 ml 0,1M Phosphatpuffer (pH 7, T= 20°C) suspendiert und mit 5 g Lipase aus Schweinepankreas (PPL, E.C. 3.1.1.3) versetzt. Durch kontinuierliche Zugabe von 1N NaOH aus einer Autobürette wird der

pH-Wert konstant auf pH 7 gehalten. Die Hydrolyse wird nach einem Verbrauch von 155 ml 1N NaOH (ca. 1 h) abgebrochen und das Gemisch kontinuierlich mit Diethylether extrahiert. Die organische Phase wird über $MgSO_4$ getrocknet, das Lösungsmittel abgezogen und der Rückstand über Kieselgel 60 (70 - 230 mesh, Diethylether/Hexan 1 : 2) chromatographiert.

Ausbeute von R-1-Hydroxy-2-O-benzylglycerin-3-acetat: 22 g (75 %)

Optischer Drehwert: $[\alpha]_D^{20}$ - 13,10° (c 2,85 in Ethanol);
$[\alpha]_D^{20}$ + 17,4°,
$[\alpha]_{365}^{20}$ + 54,9° (c 1,85 Chloroform + 1 % Ethanol);

Optische Reinheit: 90 % e.e.

4. 50 m Mol (13,3 g) 2-O-Benzylglycerin-1,3-diacetat werden in 100 ml 0,1 M Phosphatpuffer (pH 7, T= 20°C) suspendiert und mit 50 mg Lipoproteinlipase (LPL, EC 3.1.1.34) versetzt. Durch kontinuierliche Zugabe von 1 N NaOH aus einer Autobürette wird der pH-Wert konstant auf pH 7 gehalten. Die Hydrolyse wird nach einem Verbrauch von 53 ml 1 N NaOH (6 h) abgebrochen und das Reaktionsgemisch kontinuierlich mit Diethylether extrahiert. Die organische Phase wird über $MgSO_4$ getrocknet, das Lösungsmittel abgezogen und der Rückstand über Kieselgel 60 (70 - 230 mesh ; Diethylether/Hexan 1 : 2) chromatographiert.

Ausbeute von R-1-Hydroxy-2-O-benzylglycerin-3-acetat: 8,4 g (75 %)

Optischer Drehwert: $[\alpha]_D^{20}$ - 13,2° (c 2,95 in Ethanol);
$[\alpha]_D^{20}$ + 17,6°,
$[\alpha]_{365}^{20}$ + 55,2° (c 1,7 Chloroform + 1 % Ethanol);

Optische Reinheit: 91 % e.e.

5. 100 m Mol (26,6 g) 2-O-Benzylglycerin-1,3-diacetat werden in 1500 ml 0,1 M Phosphatpuffer (pH 7, T= 20°C) suspendiert und mit 50 mg Lipoproteinlipase (LPL, EC 3.1.1.34) versetzt. Durch kontinuierliche Zugabe von 1 N NaOH aus einer Autobürette wird der pH-Wert konstant auf pH 7 gehalten. Die Hydrolyse wird nach einem Verbrauch von 110,7 ml 1 N NaOH (8 h) abgebrochen und das Reaktionsgemisch kontinuierlich mit Diethylether extrahiert. Die organische Phase wird über $MgSO_4$ getrocknet, das Lösungsmittel abgezogen und der Rückstand über Kieselgel 60 (70 - 230 mesh, Diethylether/Petrolether 5 : 1) chromatographiert. Im Gegensatz zu Beispiel 4 war kein 2-O-Benzylglycerin-1,3-diacetat mehr nachzuweisen.

Ausbeute von R-1-Hydroxy-2-O-benzylglycerin-3-acetat: 17 g (77 %);
Optischer Drehwert: $[\alpha]_D^{20}$ +16,8°,
$[\alpha]_{365}^{20}$ + 52,2° (c 1,93 Chloroform + 1 % Ethanol);
Optische Reinheit: 85 % e.e.

6. Herstellung von (R)-1-O-Acetyl-2-O-benzylglycerin 3-O-(2-tetrahydropyranyl)ether (I) und (S)-2-O-Benzylglycerin-3-O-(2-tetrahydropyranyl)-ether (Gemisch der Diastereomeren)

5,2 g (23,2 m Mol) R-1-Hydroxy-2-O-benzylglycerin-3-acetat werden in 30 ml $H_2Cl_2$ gelöst und mit 5 ml Dihydropyran sowie 100 mg p-Toluolsulfonsäure über Nacht gerührt. Das Reaktionsgemisch wird mit gesättigter $NaHCO_3$-Lösung gewaschen, die organische Phase mit $MgSO_4$ getrocknet und einrotiert. Nach Chromatographie an Kieselgel (Diethylether/Petrolether 1 : 2) erhält man 6,72 g (94 %) eines Produktes (I) $[\alpha]_D^{20}$ - 0,57°; $[\alpha]_{365}^{20}$ - 2° (c 1,4 Chloroform + 1 % Ethanol). Das so erhaltene Material

wurde in 50 ml Methanol gelöst und mit 2 g $K_2CO_3$ 5 Stunden gerührt. Nach dem Einrotieren der Lösung wird in 100 ml Diethylether aufgenommen, über Kieselgel filtriert und einrotiert. Man erhält 5,5 g (95 %) 2-O-Benzylglycerin-3-O-(2-tetrahydropyranyl)-ether.

Optischer Drehwert: $[\alpha]_D^{20}$ - 1°,
$[\alpha]_{365}^{20}$ - 3,2° (c 2,05 Chloroform + 1 % Ethanol).

7. Herstellung von (S)-1-Acetoxy-2-O-Benzylglycerin-3-O-tosylat

8 g (35,7 m Mol) R-1-Hydroxy-2-O-benzylglycerin-3-acetat werden in 60 ml absolutem Tetrachlorkohlenstoff gelöst und mit 20 ml absolutem Pyridin sowie 6,9 g Toluolsulfonsäurechlorid versetzt. Die Mischung wird 24 h bei Raumtemperatur gerührt. Anschließend werden 200 ml $H_2O$ zugegeben. Die organische Phase wird abgetrennt und die wäßrige Phase mit 2 x 100 ml Diethylether extrahiert. Die vereinigten organischen Phasen werden mit 2 x 50 ml 2 N HCl und 2 x 50 ml $NaHCO_3$-Lösung gewaschen. Man trocknet über $MgSO_4$ und zieht das Lösungsmittel ab (Rohausbeute 12,3 g). Nach der Säulenchromatographie (Diethylether/Hexan 1 : 1) erhält man 10,65 g (78 %) (S)-1-Acetoxy-2-O-Benzylglycerin-3-O-tosylat:

Optischer Drehwert: $[\alpha]_D^{20}$ - 16,7°,
$[\alpha]_{365}^{20}$ - 52° (c 1,14 Chloroform + 1 % Ethanol);

Optische Reinheit: 91 % e.e.

Herstellung von (S)-2-O-Benzylglycerin-1-O-tosylat

10,65 g (28 m Mol)S-1-Acetoxy-2-O-Benzylglycerin-3-O-tosylat werden in einem Gemisch aus 100 ml 1 N HCl und 100 ml Aceton 24 h bei Raumtemperatur gerührt (DC-Kontrolle). Anschließend wird festes $NaHCO_3$ bis zur

Neutralisierung zugegeben. Nach dem Abfiltrieren von ungelöstem $NaHCO_3$ wird mit 3 x 300 ml Diethylether extrahiert und die vereinigte organische Phase über $MgSO_4$ getrocknet. Nach Abziehen des Lösungsmittels verbleiben 10,75 g Rohprodukt, dessen Chromatographie an Kieselgel (Diethylether/Hexan 3 : 1) 8,65 g (90 %) (S)-2-O-Benzylglycerin-1-O-tosylat liefert:
Optischer Drehwert: $[\alpha]_D^{20}$ - 33°,
$[\alpha]_{365}^{20}$ - 116° (c 1,78, Chloroform + 1 % Ethanol).

Herstellung von (S)-Glycerin-1-O-tosylat

6 g (24,4 mMol) (S)-2-O-Benzylglycerin-1-O-tosylat werden in 150 ml destilliertem Tetrahydrofuran gelöst und unter Zugabe von 300 mg Palladium/Kohle (10 %) bei Raumtemperatur und Normaldruck 24 h unter Rühren hydriert. Der Katalysator wird abfiltriert mit Tetrahydrofuran gewaschen. Das Lösungsmittel wird im Vakuum entfernt, der Rückstand in 100 ml Diethylether aufgenommen und über eine kurze Kieselgelsäule filtriert.

Nach dem Entfernen des Lösungsmittels erhält man 4 g (91 %) (S)-Glycerin-1-O-tosylat:
Optischer Drehwert: $[\alpha]_D^{25}$ - 8,41°,
$[\alpha]_{365}^{25}$ + 26,1° (c= 1,028 in Methanol)

Die Umkristallisation aus Diethylether/n-Hexan (1 : 2) ergibt 3,2 g (73 %) (S)-Glycerin-1-O-tosylat als farblose Nadeln:
Schmp. 61,5 - 62°C.
Optischer Drehwert: $[\alpha]_D^{25}$ - 9,45°,
$[\alpha]_D^{25}$ + 29° (c= 0,75 in Methanol).

8. Herstellung von (S)-1-Acetoxy-2-0-benzylglycerin-3-0-(diphenyl-t-butyl-silyl)-ether

3,0 g (13,4 m Mol) R-1-Hydroxy-2-0-benzylglycerin-3-acetat werden in 50 ml absolutem Tetrachlorkohlenstoff gelöst, mit 10 ml absolutem Pyridin, 0,5 g Dimethylaminopyridin und 3,7 g Diphenyl-tertiär-butylsilylchlorid versetzt und 26 h bei Raumtemperatur gerührt. Anschließend werden 200 ml $H_2O$ zugegeben, die organische Phase abgetrennt und die wäßrige Phase mit 2 x 100 ml Diethylether extrahiert. Die vereinigten organischen Phasen werden mit 2 x 50 ml 2 N HCl und danach 2 x 50 ml $NaHCO_3$-Lösung gewaschen. Man trocknet über $MgSO_4$ und zieht das Lösungsmittel ab (Rohausbeute 5,6 g). Nach Chromatographie an Kieselgel (Diethylether/Hexan 5 : 2) erhält man 4,5 g (S)-1-Acetoxy-2-0-benzylglycerin-3-0-(diphenyl-t-butyl-silyl)-ether (75 %):

Optischer Drehwert: $[\alpha]_D^{20}$ - 6,3°,
$[\alpha]_{365}^{20}$ - 20° (c 2,29 Chloroform + 1 % Ethanol).

Herstellung von (S)-2-0-Benzyl-1-0-(diphenyl-t-butylsilyl)-ether

4,5 g (9,7 m Mol) (S)-1-Acetoxy-2-0-benzylglycerin-3-0-(diphenyl-t-butyl-silyl)-ether werden in 60 ml Methanol gelöst und mit 200 mg $K_2CO_3$ versetzt. Die Mischung wird 18 h bei Raumtemperatur gerührt, Methanol abgezogen und der Rückstand in Diethylether aufgenommen. Die organische Phase wird über wenig Kieselgel filtriert. Nach dem Abdestillieren von Diethylether wird über Kieselgel chromatographiert (Laufmittel Diethylether/Hexan 2 :1). Man erhält 1,73 g (42 %) (S)-2-0-Benzyl-1-0-(diphenylt-butylsilyl)ether:

Optischer Drehwert: $[\alpha]_D^{20}$ - 12,6°, $[\alpha]_{365}^{20}$ - 40,5° (c 1,02 Chloroform + 1 % Ethanol).

2,17 g (48 %) (S)-1-Acetoxy-2-O-benzylglycerin-3-O-(diphenyl-butyl-silyl)-ether werden nicht umgesetzt.

9. Herstellung von (R)-1-Acetoxy-2-O-benzyl-3-O-tritylglycerin

5 g (22,3 m Mol) R-1-Hydroxy-2-O-benzylglycerin-3-acetat werden in 60 ml absolutem Tetrachlorkohlenstoff gelöst und mit 10 ml absolutem Pyridin sowie 500 mg Dimethylaminopyridin versetzt. Man gibt 6,2 g Tritylchlorid zu und rührt 48 h bei Raumtemperatur. Dann werden 200 ml $H_2O$ zugegeben, die organische Phase abgetrennt und die wäßrige Phase mit 2 x 250 ml Diethylether extrahiert. Die vereinigten organischen Phasen werden über $MgSO_4$ getrocknet und das Lösungsmittel abgezogen (Rohausbeute 9,23 g).

Säulenchromatographie (Diethylether/Hexan 1 : 2) lieferte 6,43 g (62 %) (R)-1-Acetoxy-2-O-benzyl-3-O-tritylglycerin:
Optischer Drehwert: $[\alpha]_D^{20}$ - 12,6°, $[\alpha]_{365}^{20}$ - 42,5° (c 1,29 Chloroform + 1 % Ethanol).

Herstellung von (S)-2-O-Benzyl-1-O-tritylglycerin

6,43 g (12,8 m Mol) (R)-1-Acetoxy-2-O-benzyl-3-O-tritylglycerin werden gemäß Beispiel 7 mit $K_2CO_3$/MeOH umgesetzt. Rohausbeute 4,2 g.

Nach der Säulenchromatographie an Kieselgel (Diethylether/Hexan 5 : 2) erhält man 3,64 g (62 %) (S)-2-O-Benzyl-1-Otritylglycerin:

Optischer Drehwert: $[\alpha]_D^{20}$ - 20,3°, $[\alpha]_{365}^{20}$ - 65° (c 1,46 Chloroform + 1 % Ethanol).

Das so erhaltene Produkt wird in siedendem Hexan gelöst und bei 4°C auskristallisiert:
Schmp. 77 - 79°C;
Optischer Drehwert: $[\alpha]_D^{20}$ - 23,6°,

$[\alpha]_{365}^{20}$ - 76° (c 1,49 Chloroform + 1 % Ethanol).

10. Herstellung von (S)-1-O-Acetyl-2-O-benzylglycerin

1,82 g 2-O-Benzylglycerin werden in 18 g absolutem Essigsäureethylester gelöst, nach Zugabe von geringen Wassermengen (0,25-1,5 % (Tab. 1)) mit 250 mg Lipase aus Pseudomonas sp. versetzt und bei Raumtemperatur gerührt. Die Reaktion wird gaschromatographisch verfolgt. Die Reaktionsmischung wird filtriert und die Lipase wieder verwendet. Das Filtrat wird vom Lösungsmittel befreit und der Rückstand an Kieselgel chromatographisch aufgetrennt (Laufmittel: Diethylether/ Petrolether 1:2)

(S)-1-O-Acetyl-2-O-benzylglycerin (Rf 0,3)
1,92 g (86 %)
Optischer Drehwert: $[\alpha]_D^{20}$ = -16,2°;

$[\alpha]_{365}^{20}$ = -51,4° c 1,76 Chloroform + 1 % Ethanol

Tabelle 1

**Alkoholyse von Essigsäureethylester in Gegenwart von 2-O-Benzylglycerin und der Lipase aus Pseudomonas sp.**

| | Wasserzugabe | | | | | | | |
|---|---|---|---|---|---|---|---|---|
| | 0,25 % | | 0,5 % | | 1,0 % | | 1,5 % | |
| Zeit (h) | % 2+ | % 3* | % 2 | % 3 | % 2 | % 3 | % 2 | % 3 |
| 17,0 | 17,4 | --- | 21,1 | --- | 29,8 | < 0,5 | 42,7 | 1,1 |
| 41,0 | 35,7 | < 0,5 | 36,9 | < 0,5 | 52,7 | 1,5 | 67,5 | 3,9 |
| 60,0 | 46,2 | 0,6 | 52,5 | 0,9 | 73,5 | 2,2 | 82,7 | 6,4 |
| 87,0 | 51,3 | 1,0 | 60,1 | 1,2 | 80,4 | 3,2 | 84,9 | 7,3 |
| 108,0 | 55,9 | 1,0 | 67,9 | 1,1 | 85,8 | 3,6 | 87,5 | 10,4 |
| 133,0 | 63,5 | 1,1 | 66,9 | 1,5 | 83,7 | 4,3 | 84,3 | 12,1 |

+(S)-1-O-Acetyl-2-O-benzylglycerin = 2
*1,3-O-Diacetyl-2-O-benzylglycerin = 3

Beispiel 11:

Entsprechend Beispiel 10 wurden 10 mmol 2-O-Benzylglycerin unter Verwendung anderer Lipasen, Zugabe von Cosolventien und verschiedener Essigsäureester umgesetzt.
Die Ergebnisse sind in Tabelle 2 aufgelistet:

Tabelle 2

| Enzym | [mg] | Essigsäureester | [ml] | Cosolvens | (S)-2 [%] | Opt. Reinheit [%ee] |
|---|---|---|---|---|---|---|
| Lipase Pseudomonas sp. | 250 | ethyl | 18 | - | 86 | 84 |
| Lipase Schweinepankreas | 500 | ethyl | 18 | - | 59 | 62 |
| Lipase Chromobacterium vis. | 250 | ethyl | 18 | - | 84 | 55 |
| Lipoprotein Lipase | 30 | ethyl | 18 | - | 63 | 40 |
| * Lipase Pseudomonas sp. | 200 | trichlorethyl | 20 | - | 37 | 85 |
| ** Lipase Pseudomonas sp. | 200 | isopropenyl | 20 | - | 5 | - |
| ** Lipase Pseudomonas sp. | 200 | " | 1 | THF (20 ml) | 12 | - |
| Lipase Pseudomonas sp. | 200 | vinyl | 1 | THF (20 ml) | 80 | 85 |
| *** Lipase Pseudomonas sp. | 200 | vinyl | 20 | - | 45 | 96 |

* kein Zusatz von Wasser

** **Umsatz zu (S)-2 nach 50 bzw. 90 Stunden**

(S)-2: S-1-O-Acetyl-2-O-benzylglycerin

*** Bildung von 50 % 1,3-O-Diacetyl-2-O-benzylglycerin

ee Enantiomerenüberschuß

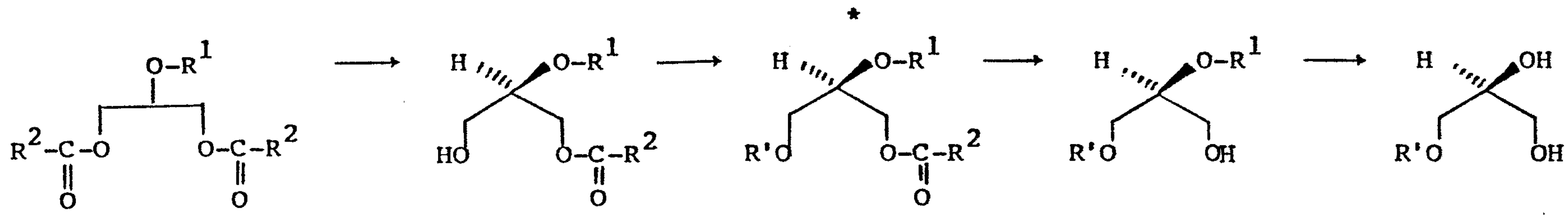

II　　I (R-Form)　　(S-Form)　　(S-Form)

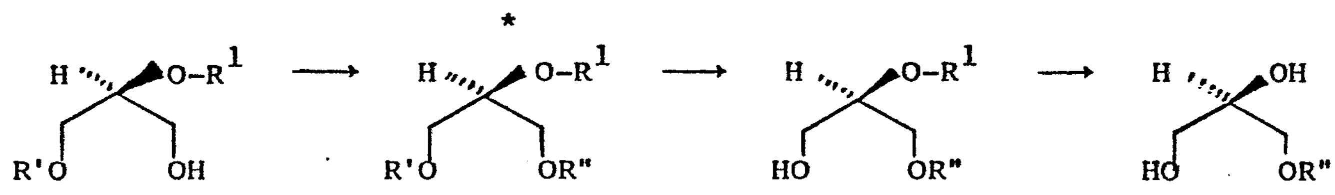

(S-Form)　　(R-Form)　　(R-Form)

* je nach Substituent R- oder S-Form

R' und R": Schutzgruppen

Patentansprüche:

1. R- und S-Enantiomere der Verbindung der allgemeinen Formel I,

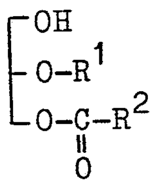

I

in der $R^1$ eine Ether-bildende Schutzgruppe und $R^2$ eine verzweigte und vorzugsweise geradkettige Alkylgruppe mit 1 bis 18 Kohlenstoffatomen darstellt, die gegebenenfalls durch Halogen, Hydroxy, Alkoxy mit 1 bis 3 Kohlenstoffatomen, Phenyl, Phenoxy und/oder Thienyl substituiert sein kann, wobei eine Phenyl- oder Phenoxygruppe substituiert sein kann durch Alkyl, Amin, Hydroxy, Halogen und Alkoxy.

2. Verbindung nach Anspruch 1, in der $R^1$ die Schutzgruppe Benzyl bzw. substituiertes Benzyl bedeutet.

3. Verfahren zur Herstellung der R- oder S-Enantiomeren der Verbindung der allgemeinen Formel I,

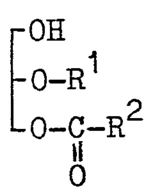

I

in der $R^1$ eine Ether-bildende Schutzgruppe und $R^2$ eine verzweigte und vorzugsweise geradkettige Alkylgruppe mit 1 bis 18 Kohlenstoffatomen darstellt, die gegebenenfalls durch Halogen, Hydroxy, Alkoxy mit 1 bis 3 Kohlenstoffatomen, Phenyl, Phenoxy und/oder Thienyl substituiert sein kann, wobei eine Phenyl- oder

Phenoxygruppe substituiert sein kann durch Alkyl, Amin, Hydroxy, Halogen und Alkoxy, dadurch gekennzeichnet, daß aus der Verbindung der allgemeinen Formel II,

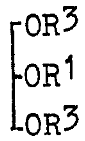 II

in der $R^1$ die obengenannte Bedeutung hat und $R^3$ Wasserstoff oder $-\overset{O}{\overset{\|}{C}}-R^2$ ist, wobei $R^2$ ebenfalls die obengenannte Bedeutung hat,

durch Inkubation mit Hydrolasen,

wenn $R^3$ Wasserstoff ist, mit der Verbindung der allgemeinen Formel $R^2-\overset{O}{\overset{\|}{C}}-OR^4$, in der $R^2$ die obengenannte Bedeutung hat und $R^4$ $C_1$ bis $C_6$ Alkyl oder Alkenyl, geradkettig oder verzweigt, das durch Halogen, Hydroxy, Alkoxy und Nitro substituiert sein kann, bedeutet, selektiv eine Hydroxylgruppe verestert wird oder,

wenn $R^3$ die Gruppe $R^2-\overset{O}{\overset{\|}{C}}-$ bedeutet, eine Estergruppe selektiv abgespalten wird.

4. Verfahren nach Anspruch 3, dadurch gekennzeichnet, daß für $R^1$ eine Benzylgruppe oder eine substituierte Benzylgruppe eingesetzt wird.

5. Verfahren nach Anspruch 3, dadurch gekennzeichnet, daß als Hydrolase eine Esterase oder Lipase eingesetzt wird.

6. Verfahren nach Anspruch 5, dadurch gekennzeichnet, daß als Hydrolase Lipase aus Schweinepankreas (E.C. 3.1.1.3) oder Lipoproteinlipase (E.C. 3.1.1.34) eingesetzt wird.

7. Verfahren nach einem oder mehreren der Ansprüche 3 bis 6, dadurch gekenzeichnet, daß die Reaktion bei 10 bis 50°C durchgeführt wird.

8. Verfahren nach einem oder mehreren der Ansprüche 3 bis 7, dadurch gekennzeichnet, daß die Reaktion bei einem pH-Wert von 5 bis 8 durchgeführt wird.

9. Verwendung der Verbindung nach Anspruch 1 als Zwischenprodukt zur Herstellung chiraler Vorstufen von pharmazeutisch aktiven Substanzen.